# EUROPEAN PATENT SPECIFICATION

(11) **EP 3 290 025 B1**
(45) Date of publication and mention of the grant of the patent: **12.02.2020**
(21) Application number: 17189128.6
(22) Date of filing: 01.09.2017
(51) Int. Cl.: A61K 9/00, A61K 31/485

(54) **STABLE INFUSION DOSAGE FORM OF MORPHINE**
STABILE INFUSIONSDOSIERFORM VON MORPHIN
FORME POSOLOGIQUE DE PERFUSION STABLE DE LA MORPHINE

(30) Priority: 02.09.2016 IN 201621030175
(43) Date of publication of application: 07.03.2018
(73) Proprietor: Sun Pharmaceutical Industries Ltd, 400063 Mumbai, Maharashtra (IN)
(72) Inventor: KUMAR, Samarth, Baroda, 390020, Gujarat (IN); KANE, Prashant, Baroda, 390020, Gujarat (IN); BHOWMICK, Subhas Balaram, Baroda, 390020, Gujarat (IN); THAKKAR, Milan Natvarbhai, Baroda, 390020 Gujarat (IN); DAVE, Kandarp Maheshkumar, Baroda, 390020, Gujarat (IN)
(74) Representative: Harrison IP Limited

(56) References cited:
- WO-A1-2014/140097
- US-A- 4 872 553
- US-A1- 2014 275 144
- Tho Nguyen-Xuan: "Stability of Morphine Sulfate in Polypropylene Infusion Bags for Use in Patient-Controlled Analgesia Pumps for Postoperative Pain Management", International Journal of Pharmaceutical Compounding, 1 January 2006 (2006-01-01), pages 69-73, XP055418593, Retrieved from the Internet: URL:http://pharmacie.hug-ge.ch/rd/publicat ions/pb_morphine_ijpc2006.pdf [retrieved on 2017-10-24]

## Description

### FIELD OF THE INVENTION

The present invention relates to a stable infusion dosage form of morphine or its pharmaceutically acceptable salt having long term stability.

### BACKGROUND OF THE INVENTION

There are many references in the literature that describe the preparation of sterile, aqueous solution of morphine or its pharmaceutically acceptable salts. For example, Xuan et al (International Journal of Pharmaceutical Compounding, Jan/Feb 2006: 10; 1; pp-69-73), describes a stable intravenous solution of *1-mg*/*mL* morphine sulfate in polypropylene bags for use in patient-controlled analgesia pumps for postoperative pain management. Three large-scale batches of *1-mg*/*mL* morphine sulfate solution filled into polypropylene bags and terminally sterilized at 120°C for 20 minutes were used in this study. The stability of the drug was monitored at 3, 6, 12, 18, 24 and 36 months after preparation in a long term study (25°C) and after 3 and 6 months in accelerated studies (30° and 40°C) using a stability-indicating high-performance liquid chromatography assay. The levels of degradation products (morphine-N-oxide and pseudomorphine) were determined. It has been reported in the reference that there was practically no loss of morphine, and impurity contents were very low. Subvisible particles were below pharmacopeial specifications. No significant change in pH was observed and water losses were minimal. The sterility of the bags was demonstrated throughout the study. Contrary to this report, in the year 2013, almost after 7 years, another publication, namely United States patent number 9072781, hereinafter referred to as '781 patent, reported the effect of steam sterilization (autoclaving) on the morphine formulation and reported that autoclaving results in formation of higher amounts of impurities as compared to formulations that are un-autoclaved.

With the mixed report on the effect of autoclaving, the inventor's checked the effect of autoclaving on stability of morphine by reproducing the example disclosed in prior art Xuan et al, wherein the morphine solution was prepared and filled in a polypropylene based infusion bag and there was no secondary overwrap or outer covering surrounding the infusion bag, and the infusion bag was subjected to autoclaving at 121°C for 15 minutes. For this, an aqueous solution of morphine having dissolved oxygen content of less than 1 ppm was prepared. The aqueous solution of morphine in volume of 100 ml was filled into a polypropylene based flexible infusion bag which was previously flushed with nitrogen. The infusion bag was sealed with a stopper. The infusion bag was not overwrapped. The naked infusion bag was terminally sterilized by moist heat sterilization in an autoclave, at a temperature of 121°C for 15 minutes. The analysis of assay (%) of morphine, % of pseudomorphine impurity, % highest unknown impurity and % total impurity in morphine solution was carried out before and after autoclaving and the data is presented below:

| Analysis Stage | Assay of Morphine (%) | % pseudomorphine | % Highest Unknown Impurity | % Total Impurity | pH |
|---|---|---|---|---|---|
| Before autoclaving | 99.20 | 0.054 | 0.019 | 0.170 | 4.73 |
| Immediately After autoclaving | 97.32 | 0.255 | 0.070 | 0.535 | 4.58 |

The chemical stability data given in above table show that there occurred significant increase in content of pseudomorphine impurity upon autoclaving and the level go beyond the desired limit of not more than 0.2 % by weight. The content of total impurities also increased significantly upon autoclaving. This indicate that the aqueous solution of morphine as per Xuan et al wherein the infusion container was not surrounded by an outer covering such as a secondary overwrap pouch cannot withstand the harsh conditions of autoclaving and becomes unstable with formation of high levels of undesired impurities.

The United States Patent Application US 2014/0275144 discloses a pre-filled syringe comprising a morphine injectable formulation. The effect of steam sterilization was studied in Example 2. The morphine formulation in a primary packaging container benefits from the addition secondary packaging that envelops the primary packaging container.

The present inventors therefore attempted to resolve the problem of morphine instability. In this context United States Patent Number 4,872,553 claims a fluid-filled plastic container comprising (a) a sealed inner envelope of plastic material filled with a fluid containing a component subject to deterioration by oxygen, said inner envelope including outlet means for changing said fluid into said inner envelope, (b) a deoxidizer, and (c) a sealed outer envelope of plastic material enclosing both said fluid-filled inner envelope and said deoxidizer wherein said deoxidizer comprises a solid deoxidizer which is enclosed in said outer envelope so that a space is left around said solid deoxidizer. This medical fluid-filled plastic container will prevent the medical fluid therein from being deteriorated even if it is subjected to steam sterilization or is stored for a long period of time.

The solid oxygen scavenger pouch when placed in the space between the container and the outer covering may come in contact with the container or outer covering during autoclaving. The placement of the oxygen scavenger pouch in the space between the inner and outer envelope may thereafter have deleterious effects. Therefore the prior art not only requires an additional step but makes the process undesirable in view of risks associated with any deleterious effects.

### SUMMARY OF THE INVENTION

With the aim of arriving at a morphine large volume infusion dosage form comprising aqueous solution of morphine that is stable on terminal sterilization by autoclaving, the inventors arrived at an infusion dosage form comprising:
(i) an aqueous solution of morphine or its pharmaceutically acceptable salt as a sole active ingredient in a concentration ranging from about 0.1 mg/ml to about 10.0 mg/ml, filled in containers in volumes ranging from about 50 ml to about 250 ml, and having dissolved oxygen content of less than 2 ppm,
(ii) the container being surrounded by an outer covering, and
(iii) an inert gas or vacuum in the space between the container and outer covering,
wherein the infusion container surrounded by an outer covering and having an inert gas in the space between the container and the outer covering is sterilized by autoclaving, wherein the infusion dosage form is stable upon autoclaving, wherein the space between the container and outer covering does not have an oxygen scavenger pouch before or during autoclaving, further wherein the container is a flexible infusion container.

The present invention does not require placing an oxygen scavenger pouch in the space between the container and outer covering before or during autoclaving.

The infusion dosage form is stable upon autoclaving meaning thereby that it can be sterilized by autoclaving, and the content of single known or unknown impurities in the solution is not more than 0.2 % by weight of morphine, and the content of total impurities is not more than 1.0 % by weight of morphine for a period of at least 6 months.

Surprisingly, aqueous solution of morphine having widely varying pH, ranging from 2.5-7.5, all remain stable under the stress conditions of autoclaving for prolonged period of time. The levels of known impurities like pseudomorphine, the highest unknown impurity and total impurities in the terminally sterilized product were within desired limits after autoclaving and further upon storage for at least 6 months at room temperature. These were found to be similar to unautoclaved sample product and lower than the marketed preparations. No loss of potency of morphine was observed in the final product.

### BRIEF DESCRIPTION OF FIGURES

Figure 1 (a) illustrate step of insertion of container i.e. an infusion bag into outer covering which is a three side sealed overwrap pouch.
Figure 2 represents the step of placement of the outer covering i.e. the three side sealed overwrap pouch containing the container which is an infusion bag into the chamber of a sealing machine.
Figure 3 represents the step of attaching nitrogen tube which supply nitrogen into the chamber when closed.
Figure 4 and Figure 5 represents closure of chamber, reduction of chamber air pressure along with flushing of nitrogen gas into the chamber through the nitrogen tube and sealing of outer covering which is the overwrap pouch.
Figure 6 represents the optional step of placement of an oxygen scavenger in the three side sealed overwrap pouch during the step of replacing the outer covering (the overwrap pouch) with a fresh outer covering i.e. another overwrap pouch after autoclaving.

### DESCRIPTION OF THE INVENTION

The term "stable upon autoclaving" as used herein means that infusion dosage form is stable in that it can be sterilized by autoclaving, and the content of single known or unknown impurities in the solution is not more than 0.2 % by weight of morphine, and the content of total impurities is not more than 1.0 % by weight of morphine. The infusion dosage form is also stable when stored at room temperature. Preferably when autoclaved and further stored at room temperature for a period of 6 months to one year, the content of single known or unknown impurities in the solution is not more than 0.2 % by weight of morphine, and the content of total impurities is not more than 1.0 % by weight of morphine. More preferably when autoclaved and further stored at room temperature for a period of 2 years, the content of single known or unknown impurities in the solution is not more than 0.2 % by weight of morphine, and the content of total impurities is not more than 1.0 % by weight of morphine.

The container of the infusion dosage form of the present invention is surrounded by an outer covering. The term 'outer covering' is intended to mean a secondary outer container into which the container comprising aqueous solution of morphine or its pharmaceutically acceptable salt is placed. The outer covering is thus an outer secondary container that surrounds the container and there is a space between the container and the outer covering i.e. the secondary outer container.

The pharmaceutically acceptable salt of morphine according to the present invention includes, but is not limited to, sulphate, hydrochloride, tartrate, hydrobromide, citrate, methobromide, lactate, bitartrate, hydroiodide, tannate, phosphate, ascorbate, acetate. The term morphine includes its free base form, salts, hydrates, polymorphs, solvates, isomers isomer salts, racemates etc. The present invention includes morphine or its salt as the sole active ingredient. Preferably, the pharmaceutically acceptable salt of morphine is morphine sulphate. In one embodiment, morphine or its pharmaceutically acceptable salt is morphine sulphate pentahydrate. Morphine or its pharmaceutically acceptable salt may be used in the aqueous solution at a concentration ranging from about 0.1 mg/ml to about 10.0 mg/ml, preferably from about 0.5 to 2.0 mg/ml. In one particularly preferred embodiment, morphine sulphate is present in the aqueous solution at a concentration of 1.0 mg/ml.

The volume capacity of each unit of infusion container may range from about 50 ml to about 500 ml. The aqueous solution of morphine or its salt may be present in the infusion container in volume ranging from about 50 ml to 500 ml, preferably from about 50 ml to 250 ml such as for example 50 ml, 75 ml, 100 ml, 120 ml, 125 ml, 140 ml, 150 ml, 160 ml, 175 ml, 180 ml, 190 ml, 200 ml, 220 ml, 225 ml, 240 ml or 250 ml. According to one preferred embodiment, the volume of aqueous solution of morphine filled in the container is 100 ml. According to another preferred embodiment, the volume of aqueous solution of morphine filled in the container is 200 ml.

The pH of the aqueous solution of morphine according to the infusion dosage form of the present invention is in the range of 2.5 to 7.5, preferably in the range of about 3.5 to 5.5, more preferably between 4.0 and 5.0, such as for example 4.05, 4.10, 4.15, 4.20, 4.25, 4.30, 4.35, 4.40, 4.45, 4.50, 4.55, 4.60, 4.65, 4.70, 4.75, 4.80, 4.85, 4.90, 4.95 or 5.0 or intermediate ranges thereof. The pH may be adjusted in the desired range by use of a suitable pH adjusting agent and/or a buffering agent, which may also help in maintaining the pH in the desired range over a period of time. The pH adjusting agent that may be used to adjust the pH in the desired range may be selected from, but not limited to, hydrochloric acid, sulfuric acid, citric acid, acetic acid, sodium hydroxide, potassium hydroxide and the like and mixtures thereof. The buffers or buffering agents that may be used to adjust and maintain the pH may be selected from a non-limiting group of pharmaceutically acceptable buffer systems such as citrate buffer, tartrate buffer, phosphate buffer, acetate buffer, lactate buffer, glycine buffer and the like or mixtures thereof.

The aqueous solution of the infusion dosage form further comprises an osmogent selected from but not limited to, sodium chloride, potassium chloride, dextrose, glucose, mannitol, sorbitol, sucrose and the like and mixtures thereof. The osmogent may be used in suitable amounts such that the solution has an osmolality in the range of about 250-500 mOsm/kg, preferably 270-330 mOsm/kg. In one preferred embodiment, the aqueous solution of morphine according to the infusion dosage form of the present invention comprises sodium chloride as the osmogent and it may be used at a concentration of 0.9% w/v. The aqueous solution of morphine according to the infusion dosage form of the present invention may further comprise other parenterally acceptable excipients.

The container used in the infusion dosage form of the present invention is a flexible infusion container, suitable for direct intravenous infusion of the aqueous solution of morphine. The container according to the present invention is preferably a single compartment container, suitable for parenteral infusion of the aqueous solution container therein. The container may be single layered or multi layered. In one embodiment, the containers have a single outlet meant for withdrawal of the aqueous solution from the container while being administered. In one embodiment, the container is a flexible infusion container which is made up of a plastic material or other polymeric material. Non limiting examples of the flexible infusion container include an infusion bag, a flexible infusion pouch, a soft bag, an infusion bottle, a film, or a plastic pre-filled syringe. The plastic or any other polymeric material of which the flexible infusion container is made, may be selected from, but not limited to, polyolefin polymers -polyethylene, polypropylene; cyclo olefin polymers, cyclo olefin copolymers, polypropylene based polyolefin polymers; polyamides, polyesters, ethylene vinyl acetate, modified polyolefin- polyethylene polymers or styrene-polyolefin based polymers and block co-polymers thereof. These flexible infusion containers may have one or more layers of plastic/polymeric materials. Preferably, the flexible container is made up of material having an oxygen transmission rate of about 100 to 1400 (ml or cm³)/(m².24 hour.atm), water vapour transmission rate of about 0.2 to 6.0 g/(m2.day) and carbon dioxide transmission rate of about 3000 to 6500 (ml or cm³)/(m².24 hour. atm).

The container filled with the aqueous solution of morphine or its salt is sealed by a stopper. In one specific embodiment, the flexible infusion container, particularly an infusion bag, may include an infusion port for example Minitulipe® infusion port which is an infusion connector having three assembled parts including a central stopper made up of chlorobutyl rubber (latex free); an upper breakable part and a bottom part, both made up of polycarbonate. In one embodiment, the flexible infusion container contains a delivery port end for insertion of an infusion set cannula/needle. In one embodiment, the flexible infusion container/bag and the delivery port connecting to the infusion needle form a system whereby during administration of the solution to the patient the vacuum created by outgress of solution is accommodated by the elasticity or flexibility of the flexible infusion bag instead of ingress of external non-sterile air. The infusion dosage form can advantageously maintain the sterility of the solution until it reaches the patient.

The outer covering according to the present invention may suitably be an overwrap pouch, or bag or film or carton that surrounds the infusion container. The outer covering is preferably made up of a material having oxygen, light and moisture barrier properties. Non limiting example of the material constituting outer covering include, aluminum, various polymers and copolymers like polyamide, ethylenevinyl alcohol copolymer etc. Aluminum based containers are preferred and include aluminium pouches, aluminium plated films, aluminium foils, aluminum laminate films, composite aluminum films co-extruded with other polymers like polyethylene, polypropylene, EVA, EMA, EAA etc. In one preferred embodiment, the outer covering is an overwrap pouch made up of composite polymer aluminium film having PET, Nylon-6, aluminium foil, and CPP (polypropylene/ethylene block copolymer) from outside to inside, the layers being either co-extruded and/or fixed using an adhesive with the other layer. In another preferred embodiment, the secondary outer container is an overwrap pouch made up of PET/ Nylon/Aluminum/Oxygen absorbing layer/Polyethylene. In another preferred embodiment, the secondary outer container is an overwrap pouch made up of PET/Nylon/Aluminum/ Oxygen absorbing layer/Polypropylene. In another preferred embodiment, the secondary outer container is an overwrap pouch made up of PET/Nylon/Aluminium/Oxygen absorbing layer/ CPP.

In one preferred embodiment, the outer covering comprises in one of its layer an oxygen scavenger or oxygen scavenging material. Non-limiting example of such oxygen scavenging materials include iron, silica, charcoal etc. Preferably the oxygen scavenging material is iron based material. In one embodiment, the oxygen scavenger may be an iron based self-reacting type or iron based water dependent type oxygen absorber.

According to the present invention, the container is overwrapped by an outer covering and the space between the container and the outer covering is occupied by an inert gas or vacuum, but without any oxygen scavenger placed in the space between the container and the outer covering. According to the present invention, the space between the container and outer covering does not have an oxygen scavenger pouch before or during autoclaving, but an oxygen scavenger pouch may be placed in the space after subjecting the infusion dosage form to autoclaving.

In one embodiment, inert gas is used to flush out or replace the air present in the space between the container and the outer covering. The inert gas may be selected from nitrogen, argon or helium.

Processes of preparing an infusion dosage form are disclosed for reference and do not form part of the claims. According to one aspect of the present invention there is provided a process of preparing an infusion dosage form, comprising the steps of:
i. preparing an aqueous solution of morphine or its pharmaceutically acceptable salt as a sole active ingredient in a concentration ranging from about 0.1 mg/ml to about 10.0 mg/ml and having dissolved oxygen content of less than 2 ppm,
ii. filling the aqueous solution of step (i) in a container in volumes ranging from about 50 ml to about 250 ml,
iii. overwrapping the container by an outer covering, and replacing the air in the space between the container and outer covering with an inert gas or vacuum,
iv. autoclaving the overwrapped filled container of step (iii), removing the outer covering after autoclaving to inspect the clarity of the aqueous solution and then placing the filled container in fresh outer covering;
v. replacing the air in the space between the container and outer covering with an inert gas or vacuum;
vi. optionally, placing an oxygen scavenger pouch in the space between the container and outer covering.
further wherein the space between the container and outer covering does not have an oxygen scavenger pouch before or during autoclaving.

According to one aspect of the present invention there is provided a process of preparing an infusion dosage form, comprising the steps of:
i. preparing an aqueous solution of morphine or its pharmaceutically acceptable salt as a sole active ingredient in a concentration ranging from about 0.1 mg/ml to about 10.0 mg/ml and having dissolved oxygen content of less than 2 ppm,
ii. filling the aqueous solution of step (i) in a container in volumes ranging from about 50 ml to about 250 ml,
iii. overwrapping the infusion container by an outer covering having an oxygen scavenger in one of its layer, and replacing the air in the space between the container and outer covering with an inert gas or vacuum,
iv. autoclaving the overwrapped filled container of step iii), removing the overwrap after autoclaving to inspect the clarity of the aqueous solution and then placing the filled container in fresh outer covering;
v. replacing the air in the space between the container and outer covering having an oxygen scavenger in one of its layer with an inert gas or vacuum.
further wherein the space between the container and outer covering does not have an oxygen scavenger pouch before or during autoclaving.

In another preferred embodiment, the present invention provides a process of preparing an infusion dosage form, comprising the steps of:
i. preparing an aqueous solution of morphine or its pharmaceutically acceptable salt as a sole active ingredient in a concentration ranging from about 0.1 mg/ml to about 10.0 mg/ml and having dissolved oxygen content of less than 2 ppm,
ii. filling the aqueous solution of step (i) in a container in volumes ranging from about 50 ml to about 250 ml,
iii. overwrapping the container by an outer covering, and replacing the air in the space between the container and outer covering with an inert gas or vacuum,
iv. autoclaving the overwrapped filled container of step iii), removing the outer covering after autoclaving to inspect the clarity of the aqueous solution and then placing the filled container in fresh outer covering;
v. replacing the air in the space between the container and outer covering with an inert gas or vacuum;
further wherein the space between the container and outer covering does not have an oxygen scavenger pouch before or during or after autoclaving.

In another preferred embodiment, the present invention provides a process of preparing an infusion dosage form, comprising the steps of:
i. preparing an aqueous solution of morphine or its pharmaceutically acceptable salt as a sole active ingredient in a concentration ranging from about 0.1 mg/ml to about 10.0 mg/ml and having dissolved oxygen content of less than 2 ppm,
ii. filling the aqueous solution of step (i) in a container in volumes ranging from about 50 ml to about 250 ml,
iii. overwrapping the container by an outer covering, and replacing the air in the space between the container and outer covering with an inert gas or vacuum,
iv. iv. autoclaving the overwrapped filled container of step iii), removing the outer covering after autoclaving to inspect the clarity of the aqueous solution and then placing the filled container in fresh outer covering;
v. replacing the air in the space between the container and outer covering with an inert gas or vacuum;
vi. placing an oxygen scavenger pouch in the space between the container and outer covering;
further wherein the space between the container and outer covering does not have an oxygen scavenger pouch before or during autoclaving.

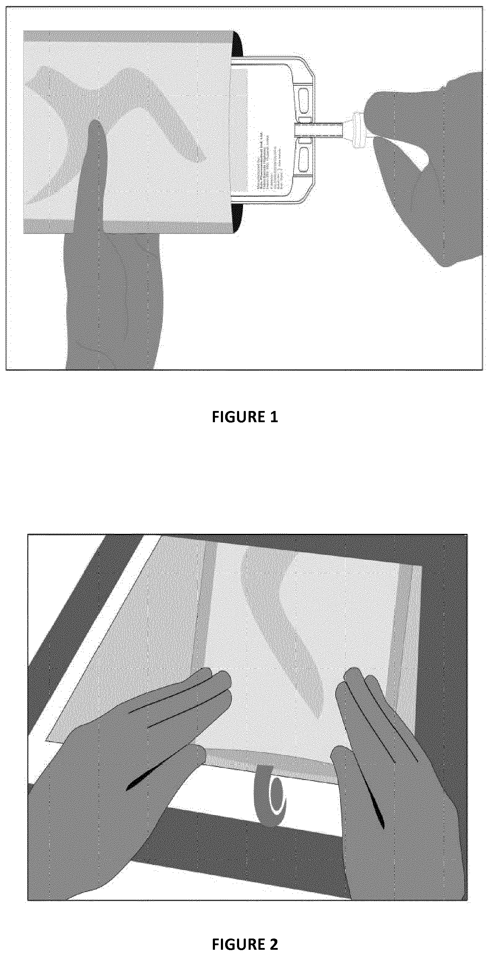

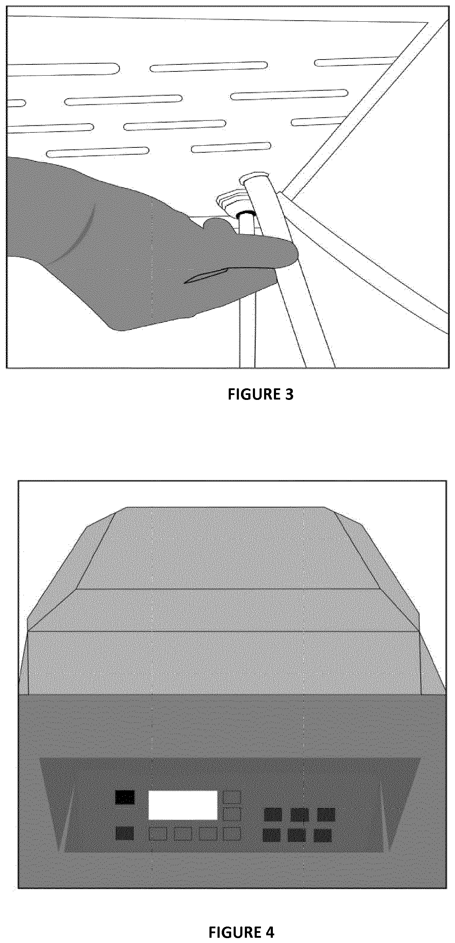

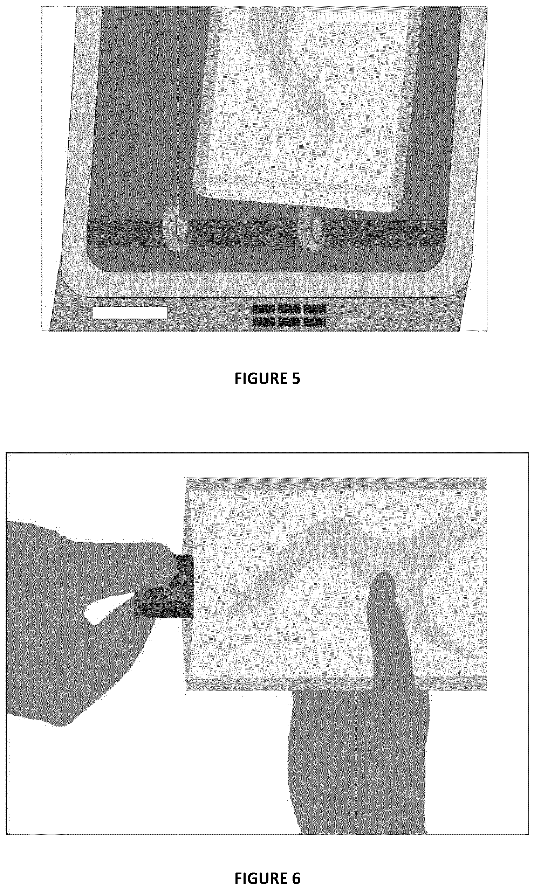

In one embodiment, the process of overwrapping the infusion container comprises the steps of inserting the filled infusion container (for eg. infusion bag) into three side sealed overwrap pouch or outer covering (Figure 1), followed by placing the three side sealed overwrap pouch containing the infusion bag (container) into the chamber of a sealing machine, such as Multivac (Figure 2). This is accompanied by step of attaching a nitrogen tube to the machine which is capable of supplying nitrogen into the chamber when closed (Figure 3). This is followed by closure of the chamber, reduction of chamber air pressure and flushing of nitrogen gas into the chamber through the nitrogen tube and sealing of overwrap pouch (Figure 4 & 5). This provides infusion container surrounded by a sealed overwrap pouch such that the space between the container and the overwrap pouch (i.e. outer covering) is occupied by the Nitrogen (inert) gas. While formulating the infusion dosage form of the present invention, the infusion container surrounded by the outer covering (sealed overwrap pouch) and having inert gas in the space between the container and the outer covering, is subjected to autoclaving.

The aqueous solution of morphine contained in the infusion container according to the present invention preferably have dissolved oxygen content of less than 2 ppm (parts per million), preferably less than 1 ppm. The aqueous solution of morphine, while formulating, is purged with an inert gas like nitrogen or argon so that the content of dissolved oxygen in the solution is less than 2 ppm (parts per million), preferably less than 1 ppm. Further, the headspace of the container, i.e. the space above the solution in the container, if present, may be replaced by an inert gas, by flushing the inert gas in the head space. Preferably, the head space in the container is less than 5% by volume.

According to the present invention, the aqueous solution of morphine or its salt contained in the container is sterile. Sterilization is achieved by moist heat sterilization or autoclaving wherein the filled and sealed container together with the outer covering and an inert gas in the space between the container and outer covering is terminally sterilized, more particularly sterilized by autoclaving. The autoclaving is preferably carried out at 121°C for 15 minutes. In one or more embodiments, the autoclaving may be carried out at temperatures varying from about 110°C to 125°C for a period of time varying from about 5 minutes to 60 minutes. In some embodiments, other sterilization techniques such as filtration sterilization, radiation sterilization etc., may be used concurrently with moist heat sterilization.

The inventors discovered that the infusion dosage form of the present invention remains stable, physically and chemically, even upon being subjected to harsh conditions of autoclaving (higher temperatures of the order of 110-130°C) and subsequent storage. There occurs substantially no degradation of morphine and the solution filled in the container show optimum storage stability at room temperature, for a period of at least 6 months. Surprisingly, aqueous solutions of morphine having varying pH, ranging from 2.5-7.5, remains stable under the stress conditions of autoclaving. The level of known impurities like pseudomorphine, the highest unknown impurity and total impurities in the terminally sterilized product remains within desired limit. The content of single known impurities like pseudomorphine, 10-alpha hydroxyl morphine, morphinone, oripavine, apomorphine, codeine sulphate or unknown impurities in the solution remains not more than 0.2 % by weight of morphine, and the content of total impurities remains not more than 1.0 % by weight of morphine, when stored at room temperature for a period of at least 6 months. Preferably, the levels are maintained within this limit for at least 2 years when stored at room temperature. Preferably, the content of pseudomorphine impurity which is a pertinent impurity, remains below 0.15 % by weight of morphine and the content of highest unknown impurity remains below 0.10 % by weight of morphine when stored at room temperature for a period of at least 6 months. The levels of impurities were found to be similar to unautoclaved solution product and lower than the marketed preparations. No loss of potency of morphine was observed in the final product.

In one specific embodiment, the present invention provides infusion dosage form with a flexible infusion bag comprising an aqueous solution of morphine or its pharmaceutically acceptable salt as a sole active ingredient in a concentration ranging from about 0.1 mg/ml to about 10.0 mg/ml, in volumes ranging from about 50 ml to about 250 ml, and having dissolved oxygen level of less than 2 ppm, wherein the flexible infusion bag is surrounded by an outer covering comprising an aluminum pouch and an oxygen scavenger in one of the layers of the outer covering and the space between the flexible infusion bag and the pouch is occupied by an inert gas or vacuum and wherein the space between the container and outer covering does not have an oxygen scavenger pouch before or during autoclaving. The infusion dosage form is stable in that the flexible infusion bag having the aqueous solution of morphine and surrounded by the outer covering, when sterilized by autoclaving, and when stored at room temperature for a period of at least 6 months, for example 1 year, 2 years or 3 years, the content of single known impurity like pseudomorphine or unknown impurities in the solution is not more than 0.2 % by weight of morphine, and the content of total impurities is not more than 1.0 % by weight of morphine.

In another specific embodiment, the present invention provides infusion dosage form comprising an aqueous solution of morphine or its pharmaceutically acceptable salt as a sole active ingredient at a concentration ranging from 1.0 mg/ml to 2.0 mg/ml, filled in containers in volumes ranging from about 100 ml to about 200 ml, and having dissolved oxygen level of less than 2 ppm and pH in the range of 4.0 to 7.5, wherein the containers is a flexible infusion bag and is surrounded by an overwrap pouch such that the space between the flexible infusion bag and the pouch is occupied by an inert gas or vacuum, wherein the infusion dosage form is sterilized by autoclaving, further wherein the space between the container and outer covering does not have an oxygen scavenger pouch before or during autoclaving and further wherein the infusion dosage form is stable upon autoclaving and upon storage at room temperature for a period of at least 6 months, for example 1 year, 2 years or 3 years, such that the content of pseudomorphine impurity or highest unknown impurity in the solution is not more than 0.2 % by weight of morphine, and the content of total impurities is not more than 1.0 % by weight of morphine.

Advantageously, since morphine is present in the aqueous solution of the current invention at lower concentration and the solution is filled in the flexible bags at higher volumes, essentially morphine remains in diluted form in the flexible bag, and as a result a potential morphine abuser will be deterred for drug abuse, since large volume of solvent will be required for extracting morphine out of the diluted aqueous solution.

In the context of this specification "comprising" is to be interpreted as "including". Aspects of the invention comprising certain elements are also intended to extend to alternative embodiments "consisting" or "consisting essentially" of the relevant elements.

Where technically appropriate, embodiments of the invention may be combined. Embodiments are described herein as comprising certain features/elements. The disclosure also extends to separate embodiments consisting or consisting essentially of said features/elements.

Hereinafter, the invention is more specifically described by way of examples. The examples are not intended to limit the scope of the invention and are merely used as illustrations.

### EXAMPLE 1

The example illustrates an infusion dosage form of morphine according to one embodiment of the present invention. This example also illustrates the following process:
i. preparing an aqueous solution of morphine or its pharmaceutically acceptable salt as a sole active ingredient in a concentration ranging from about 0.1 mg/ml to about 10.0 mg/ml and having dissolved oxygen content of less than 2 ppm,
ii. filling the aqueous solution of step (i) in a container in volumes ranging from about 50 ml to about 250 ml,
iii. overwrapping the infusion container by an outer covering having an oxygen scavenger in one of its layer, and replacing the air in the space between the container and outer covering with an inert gas or vacuum,
iv. autoclaving the overwrapped filled container of step iii), removing the overwrap after autoclaving to inspect the clarity of the aqueous solution and then placing the filled container in fresh outer covering;
v. replacing the air in the space between the container and outer covering having an oxygen scavenger in one of its layer with an inert gas or vacuum,
further wherein the space between the container and outer covering does not have an oxygen scavenger pouch before or during autoclaving.

**Table 1: Composition of aqueous solution of morphine**

| Ingredients | Concentration (mg/ml) |
|---|---|
| Morphine sulphate pentahydrate | 1.0 |
| Sodium Chloride | 9.0 |
| Hydrochloric acid/sulfuric acid/sodium hydroxide | q.s. to adjust pH to 4.0 to 5.0 |
| Water for Injection | q.s. |

Method of preparation: Water for injection was taken in a vessel and nitrogen gas was purged in it for about 20 minutes to achieve dissolved oxygen content of less than 1 ppm. Sodium chloride was added to water and it was stirred until dissolution. To this, morphine sulfate pentahydrate was added along with stirring and was dissolved. The pH was adjusted with 1% sulfuric acid / hydrochloric acid to a pH of 4.0 to 5.0. The volume was made up with water for injection along with stirring. The nitrogen purging was carried out during the process to keep the dissolved oxygen content less than 1 ppm. The aqueous solution of morphine in volume of 100 ml was filled into container i.e. a flexible infusion bag which was previously flushed with nitrogen. The infusion bag was then sealed with a stopper and was overwrapped with an outer covering along with special grade nitrogen gas (having <1 PPM oxygen) such that the space between the infusion bag and outer covering is occupied with nitrogen gas. The outer covering used was multilayered and includes an oxygen scavenger in one of its layers. The configuration of outer covering was as follows; layers from outside to inside made up of Polyethylene terephthylate/Nylon/Aluminium/Oxygen absorbing layer/Polypropylene. The space between the container and the outer covering did not include a pouch of oxygen scavenger. The infusion bag overwrapped with the outer covering i.e. overwrap pouch was terminally sterilized by moist heat sterilization in an autoclave, at a temperature of 121°C for 15 minutes. The overwrap pouch was replaced by a fresh overwrap pouch and the space between the infusion bag and overwrap pouch having an oxygen scavenger in one of its layers was occupied with nitrogen gas and not having any oxygen scavenger in the space between the container and the outer covering i.e. the overwrap pouch.

In this example, the container was a cycloolefin based flexible infusion bag comprising an inner layer (which remains in contact with the aqueous solution) made up of a cycloolefin polymer, a middle layer made up of linear low density polyethylene polymer and an outer layer made up of low density polyethylene polymer. This infusion bag has a water vapour transmission rate of 2 g (m².day) when measured at (40 °C/90% relative humidity); oxygen transmission rate of 570 ml/(m².24 hour.atm) when measured at (23 °C/0% relative humidity) and carbon dioxide transmission rate of 3400 ml/(m².24hour.atm) when measured at 23°C/0% relative humidity.

Other batches were prepared similarly using different type of infusion bag. In one such batch, the infusion bag used was a multi-layered, polypropylene based flexible infusion bag having a water vapour transmission rate of 0.62 g (m2.day) when measured at 23 °C/60% relative humidity; oxygen permeability of 1110 ml/(m².24hour.atm) when measured at 23 °C/40% relative humidity and carbon dioxide transmission rate of 5149 ml/(m².24hour.atm). In one such batch, the infusion bag used was a multi-layered, polyoleifin flexible infusion bag comprising a multilayer polyolefin film having a water vapour transmission rate of 5.0 g (m².day) when measured at 38°C/100% relative humidity; oxygen transmission rate of 1315 cm³/(m².24hour.atm) when measured at 73°F/0% relative humidity and carbon dioxide transmission rate of 3945 cm³/(m².24hour.atm).

### EXAMPLE 2

The example illustrates an infusion dosage form of morphine according to another embodiment of the present invention.

This example also illustrates the following process:
i. preparing an aqueous solution of morphine or its pharmaceutically acceptable salt as a sole active ingredient in a concentration ranging from about 0.1 mg/ml to about 10.0 mg/ml and having dissolved oxygen content of less than 2 ppm,
ii. filling the aqueous solution of step (i) in a container in volumes ranging from about 50 ml to about 250 ml,
iii. overwrapping the container by an outer covering, and replacing the air in the space between the container and outer covering with an inert gas or vacuum,
iv. iv. autoclaving the overwrapped filled container of step iii), removing the outer covering after autoclaving to inspect the clarity of the aqueous solution and then placing the filled container in fresh outer covering;
v. replacing the air in the space between the container and outer covering with an inert gas or vacuum;
further wherein the space between the container and outer covering does not have an oxygen scavenger pouch before or during or after autoclaving.

Similar to example 1, an aqueous solution of morphine was prepared and filled into infusion container i.e. a flexible infusion bag which was previously flushed with nitrogen. The infusion bag was sealed with a stopper. Subsequently, the infusion bag was overwrapped with an outer covering along with special grade nitrogen gas (having <1 PPM oxygen) such that the space between the infusion bag and outer covering is occupied with nitrogen gas. The outer covering used herein was a multilayered overwrap pouch having layers from outside to inside made up of Polyethylene terephthylate/Nylon-6/Aluminium/CPP (polypropylene/ethylene block copolymer). The multilayered overwrap pouch did not have an oxygen scavenger material in one of its layers. Further, the space between the container and the outer covering did not include a pouch of oxygen scavenger. The infusion bag overwrapped with the overwrap pouch was terminally sterilized by moist heat sterilization in an autoclave, at a temperature of 121°C for 15 minutes. The overwrap pouch was replaced by a fresh overwrap pouch same as before and the space between the infusion bag and overwrap pouch was occupied with nitrogen gas. The space between the infusion bag and the overwrap pouch did not include a pouch of oxygen scavenger.

The infusion bag used herein was a cyclooleifin based flexible infusion bag comprising an inner layer (which remains in contact with the aqueous solution) made up of a cycloolefin polymer, a middle layer made up of linear low density polyethylene polymer and an outer layer made up of low density polyethylene polymer. The infusion bag has a water vapour transmission rate of 2 g (m².day) when measured at (40 °C/90% relative humidity); oxygen transmission rate of 570 ml/(m².24 hour.atm) when measured at (23 °C/0% relative humidity) and carbon dioxide transmission rate of 3400 ml/(m².24hour.atm) when measured at 23°C/0% relative humidity.

### EXAMPLE 3

The example illustrates an infusion dosage form of morphine according to another embodiment of the present invention.

This example also illustrates the following process:
i. preparing an aqueous solution of morphine or its pharmaceutically acceptable salt as a sole active ingredient in a concentration ranging from about 0.1 mg/ml to about 10.0 mg/ml and having dissolved oxygen content of less than 2 ppm,
ii. filling the aqueous solution of step (i) in a container in volumes ranging from about 50 ml to about 250 ml,
iii. overwrapping the container by an outer covering, and replacing the air in the space between the container and outer covering with an inert gas or vacuum,
iv. iv. autoclaving the overwrapped filled container of step iii), removing the outer covering after autoclaving to inspect the clarity of the aqueous solution and then placing the filled container in fresh outer covering;
v. replacing the air in the space between the container and outer covering with an inert gas or vacuum;
vi. placing an oxygen scavenger pouch in the space between the container and outer covering;
further wherein the space between the container and outer covering does not have an oxygen scavenger pouch before or during autoclaving.

An aqueous solution of morphine similar to example 1 (Table 1) was prepared. Water for injection was taken in a vessel and nitrogen gas was purged in it for about 20 minutes to achieve dissolved oxygen content of less than 1 ppm. Sodium chloride was added to water and it was stirred until dissolution. To this, morphine sulfate pentahydrate was added along with stirring and was dissolved. The pH was adjusted with 1% sulfuric acid / hydrochloric acid to a pH of 4.0 to 5.0. The volume was made up with water for injection along with stirring. The nitrogen purging was carried out during the process to keep the dissolved oxygen content less than 1 ppm. The aqueous solution of morphine in volume of 100 ml was filled into infusion container i.e. a flexible infusion bag which was previously flushed with nitrogen. The infusion bag was sealed with a stopper. Subsequently, the infusion bag was overwrapped with an outer covering along with special grade nitrogen gas (having <1 PPM oxygen) such that the space between the infusion bag and outer covering is occupied with nitrogen gas. The outer covering used herein was a multilayered overwrap pouch similar to Example 2, having layers from outside to inside made up of Polyethylene terephthylate/Nylon-6/Aluminium/CPP (polypropylene/ethylene block copolymer). The multilayered overwrap pouch did not have an oxygen scavenger material in one of its layers. Further, the space between the container and the outer covering did not include a pouch of oxygen scavenger at this stage. The overwrapped infusion bag was terminally sterilized by moist heat sterilization in an autoclave, at a temperature of 121°C for 15 minutes. The overwrap pouch was replaced by a fresh overwrap pouch same as before and the space between the infusion bag and overwrap pouch was occupied with nitrogen gas. An oxygen scavenger pouch was also placed in the space between the infusion bag and overwrap pouch.

The infusion bag used herein was a multi-layered, polyoleifin flexible infusion bag comprising a multilayer polyolefin film having a water vapour transmission rate of 5.0 g (m².day) when measured at 38°C/100% relative humidity; oxygen transmission rate of 1315 cm³/(m².24hour.atm) when measured at 73°F/0% relative humidity and carbon dioxide transmission rate of 3945 cm³/(m².24hour.atm).

Several infusion dosage forms of morphine were prepared according to the process as illustrated by examples 1, 2 and 3 and were tested for storage stability at room temperature (25°C, 40 % relative humidity) as well as at accelerated stability testing condition of 40°C; 25% relative humidity. It was observed that the aqueous solution of morphine remained physically and chemically stable after autoclaving and upon subsequent storage at room temperature (25°C; 40% relative humidity) as well as at accelerated condition of 40°C/25% relative humidity, for at least 6 months. There occurred no drop in the assay of morphine and no substantial increase in the impurities level, and the values remained within the desired specified limits. The total impurities remained well within the desired limit of 'not more than 1.0 %' by weight of morphine. The pertinent known impurity -pseudomorphine remain within the limit of 'not more than 0.2%' by weight of morphine. The single highest unknown impurity in the solution remained within the limit of 'not more than 0.2%' by weight of morphine. The solution remained physically stable, such that no precipitation or crystallization or color change took place upon autoclaving and upon storage.

## Claims

1. An infusion dosage form comprising
i. an aqueous solution of morphine or its pharmaceutically acceptable salt as a sole active ingredient in a concentration ranging from about 0.1 mg/ml to about 10.0 mg/ml, filled in containers in volumes ranging from about 50 ml to about 250 ml, and having dissolved oxygen content of less than 2 ppm,
ii. the container being surrounded by an outer covering, and
iii. an inert gas or vacuum in the space between the container and outer covering, wherein the infusion container surrounded by an outer covering and having an inert gas in the space between the container and the outer covering is sterilized by autoclaving, wherein the infusion dosage form is stable upon autoclaving, wherein the space between the container and outer covering does not have an oxygen scavenger pouch before or during autoclaving, further wherein the container is a flexible infusion container.

2. The infusion dosage form as claimed in claim 1, wherein the solution has a pH in the range of 4.0 to 7.5.

3. The infusion dosage form as claimed in claim 1, wherein the head space in the container is less than 5 % by volume.

4. The infusion dosage form as claimed in claim 1, wherein the flexible infusion container is selected from an infusion bag, a flexible infusion pouch, a soft bag, an infusion bottle, a film, or a plastic pre-filled syringe.

5. The infusion dosage form as claimed in claim 4, wherein the flexible infusion container is made up of material having an oxygen transmission rate in the range of about 100 to 1400 cm³/(m².24 hour.atm), water vapour transmission rate in the range of about 0.2 to 6.0 g/(m².day) and carbon dioxide transmission rate in the range of about 3000 to 6500 cm³/(m².24 hour. atm).

6. The infusion dosage form as claimed in claim 1, wherein the outer covering is selected from an overwrap bag or overwrap pouch or film.

7. The infusion dosage form as claimed in claim 6, wherein the outer covering is made up of a material comprising aluminium.

8. The infusion dosage form as claimed in claim 1, wherein the outer covering comprises a layer having an oxygen scavenger.

9. The infusion dosage form as claimed in claim 1, wherein the infusion dosage form is sterilized by autoclaving at a temperature ranging from about 105°C to 130°C for a period of 10 minutes to 60 minutes.

10. The infusion dosage form as claimed in claim 1, wherein the solution comprises an osmogent selected from sodium chloride, dextrose, or glucose.

## Patentansprüche

1. Eine Infusionsdosierungsform, umfassend
i. eine wässrige Lösung von Morphin oder seinem pharmazeutisch verträglichen Salz als alleiniger Wirkstoff in einer Konzentration von etwa 0,1 mg/ml bis etwa 10,0 mg/ml, abgefüllt in Behältern mit einem Volumen von etwa 50 ml bis etwa 250 ml und mit einem Gehalt an gelöstem Sauerstoff von weniger als 2 ppm,
ii. wobei der Behälter von einer äußeren Umhüllung umgeben ist, und
iii. ein Inertgas oder Vakuum in dem Raum zwischen dem Behälter und der äußeren Umhüllung, wobei der von einer äußeren Umhüllung umgebene und ein Inertgas in dem Raum zwischen dem Behälter und der äußeren Umhüllung aufweisende Infusionsbehälter durch Autoklavieren sterilisiert wird, wobei die Infusionsdosierungsform beim Autoklavieren stabil ist, wobei der Raum zwischen dem Behälter und der äußeren Umhüllung vor oder während des Autoklavierens keinen Sauerstoffbinder aufweist, wobei der Behälter ferner ein flexibler Infusionsbehälter ist.

2. Infusionsdosierungsform nach Anspruch 1, wobei die Lösung einen pH-Wert im Bereich von 4,0 bis 7,5 aufweist.

3. Infusionsdosierungsform nach Anspruch 1, wobei der Kopfraum im Behälter weniger als 5 % Volumenanteil beträgt.

4. Infusionsdosierungsform nach Anspruch 1, wobei der flexible Infusionsbehälter ausgewählt ist aus einer Infusionstasche, einem flexiblen Infusionsbeutel, einer weichen Tasche, einer Infusionsflasche, einer Folie oder einer vorgefüllten Kunststoffspritze.

5. Infusionsdosierungsform nach Anspruch 4, wobei der flexible Infusionsbehälter aus einem Material mit einer Sauerstoffdurchlässigkeitsrate im Bereich von etwa 100 bis 1400 cm³/(m².24 h.atm), einer Wasserdampfdurchlässigkeitsrate im Bereich von etwa 0,2 bis 6,0 g/(m².Tag) und einer Kohlendioxiddurchlässigkeitsrate im Bereich von etwa 3000 bis 6500 cm³/(m².24 h.atm) besteht.

6. Infusionsdosierungsform nach Anspruch 1, wobei die äußere Umhüllung aus einer Umwicklungstasche oder einem Umwicklungsbeutel oder einer Folie ausgewählt ist.

7. Infusionsdosierungsform nach Anspruch 6, wobei die äußere Hülle aus einem aluminiumhaltigen Material besteht.

8. Infusionsdosierungsform nach Anspruch 1, wobei die äußere Hülle eine Schicht mit einem Sauerstoffbinder umfasst.

9. Infusionsdosierungsform nach Anspruch 1, wobei die Infusionsdosierungsform durch Autoklavieren bei einer Temperatur im Bereich von etwa 105 °C bis 130 °C über einen Zeitraum von 10 Minuten bis 60 Minuten sterilisiert wird.

10. Infusionsdosierungsform nach Anspruch 1, wobei die Lösung einen osmotischen Wirkstoff umfasst, ausgewählt aus Natriumchlorid, Dextrose oder Glucose.

## Revendications

1. Forme posologique de perfusion comprenant
i. une solution aqueuse de morphine ou son sel pharmaceutiquement acceptable comme seul principe actif en une concentration allant d'environ 0,1 mg/ml à environ 10,0 mg/ml, remplie dans des récipients en volumes allant d'environ 50 ml à environ 250 ml, et présentant une teneur en oxygène dissous inférieure à 2 ppm,
ii. le récipient entouré d'une enveloppe extérieure, et
iii. un gaz inerte ou un vide dans l'espace entre le récipient et l'enveloppe externe, ledit récipient de perfusion entouré d'une enveloppe externe et comportant un gaz inerte dans l'espace entre le récipient et l'enveloppe externe étant stérilisé par autoclavage, ladite forme posologique de perfusion étant stable lors de l'autoclavage, ledit espace entre le récipient et l'enveloppe externe ne comportant pas de poche de désoxygénant avant ou pendant l'autoclavage, en outre ledit récipient étant un récipient de perfusion souple.

2. Forme posologique de perfusion selon la revendication 1, ladite solution ayant un pH dans la plage de 4,0 à 7,5.

3. Forme posologique de perfusion selon la revendication 1, l'espace de tête dans le récipient représentant moins de 5 % en volume.

4. Forme posologique de perfusion selon la revendication 1, ledit récipient de perfusion souple étant choisi parmi un sachet de perfusion, une poche de perfusion souple, un sachet souple, un flacon de perfusion, un film ou une seringue préremplie en plastique.

5. Forme posologique de perfusion selon la revendication 4, ledit récipient de perfusion souple étant constitué d'un matériau présentant un taux de transmission de l'oxygène dans la plage d'environ 100 à 1400 cm³/(m².24 heures.atm), un taux de transmission de la vapeur d'eau dans la plage d'environ 0,2 à 6,0 g/(m².jour) et un taux de transmission du dioxyde de carbone dans la plage d'environ 3000 à 6500 cm³/(m².24 heures.atm).

6. Forme posologique de perfusion selon la revendication 1, ladite enveloppe externe étant choisie parmi un sachet de suremballage ou une poche de suremballage ou un film.

7. Forme posologique de perfusion selon la revendication 6, ladite enveloppe externe étant constituée d'un matériau comprenant de l'aluminium.

8. Forme posologique de perfusion selon la revendication 1, ladite enveloppe externe comprenant une couche comportant un désoxygénant.

9. Forme posologique de perfusion selon la revendication 1, ladite forme posologique de perfusion étant stérilisée par autoclavage à une température allant d'environ 105°C à 130°C pendant une période de 10 minutes à 60 minutes.

10. Forme posologique de perfusion selon la revendication 1, ladite solution comprenant un agent osmotique choisi parmi le chlorure de sodium, le dextrose ou le glucose.
